# EUROPEAN PATENT SPECIFICATION

(11) **EP 4 282 742 B1**
(45) Date of publication and mention of the grant of the patent: **21.10.2026**
(21) Application number: 22742083.3
(22) Date of filing: 14.01.2022
(51) Int. Cl.: B62K 15/00, F16C 11/04, F16C 11/10

(54) **FOLDING JOINT**
FALTGELENK
CHARNIÈRE PLIANTE

(30) Priority: 19.01.2021 CN 202120138265 U
(43) Date of publication of application: 29.11.2023
(73) Proprietor: Shenzhen Meidahang Technology Co., Ltd., Shenzhen, Guangdong 518100 (CN)
(72) Inventor: MO, Yiping, Shenzhen, Guangdong 518100 (CN); HON, David Tak Wei, Shenzhen, Guangdong 518100 (CN); LIU, Guoping, Shenzhen, Guangdong 518100 (CN)
(74) Representative: Reddie & Grose LLP
(86) International application number: PCT/CN2022/071972
(87) International publication number: WO 2022/156604

(56) References cited:
- EP-A1- 3 584 148
- CN-A- 1 406 808
- CN-A- 102 806 967
- CN-U- 212 099 205
- CN-U- 214 451 577
- CN-Y- 2 494 788
- JP-A- 2000 085 671
- JP-A- 2002 037 171
- US-A- 5 337 609
- US-A1- 2019 256 165

## Description

### TECHNICAL FIELD

The present utility model relates to the field of folding technology, in particular to a folding joint.

### BACKGROUND

As a means of transportation, bicycle plays an important role in people's daily life. For the convenience of portability, folding bicycles appear in people's lives. And in order to realize the switching between a folding state and a non-folding state of the folding bicycle, a folding joint is needed.

Existing folding joints generally include two connecting rods and two joints connecting two sections of vehicle pipes respectively. An end of each of the two joints is hinged, and a locking device is arranged to externally or internally secure two connecting plates that are close together, so that they cannot be opened. Since the joints are used on the frame, they are subjected to a lot of force during riding bumps. The folding joints must not only ensure sufficient rigidity when they are closed and fastened, but also make the opening and closing operations of the joints quick and convenient. Therefore, the locking device has to be designed thicker and has a complicated structure, and the manufacturing cost is high.

US patent application with publication number US2019/256165 discloses an elliptical bicycle comprising a structural frame including two stringers in opposing arcs, one upper and the other lower, a front wheel and a rear wheel driven by a crankset from its support platforms, as well as a steering box formed by fork, steering tube and handlebar. The elliptical bicycle has multiple hinges. The structural frame is provided with a set of main hinges, each applied to a bipartite stringer having an articulation at an approximate angle of up to 170°. A secondary hinge is applied in a bipartite steering tube of the handlebar having an articulation at an approximate angle of up to 180°. A set of tertiary hinges is applied between a crankset and support platforms of the user's feet, each of them having an articulation at an approximate angle of up to 90° upwards.

Japanese patent application with publication number JP2000085671 discloses a fastening device for a folding bicycle. The fastening device is fastened by fastening a joint plate in a closing state, defining a folding portion, with a first and second fasteners of a clamp, and fixing fastening by the first and second fasteners in operation of a fastening shaft.

US patent number US5337609 discloses a handlebar structure for a collapsible bicycle which mainly uses a locking mechanism to clamp and lock the upper tube and the lower tube of the bicycle together. The locking mechanism comprises a bolt, a pressing lever and a U-shaped clamping member. The bolt feeds through the pivotal connection of the upper tube and the lower tube, with its front end being secured to the U-shaped clamping member and its rear end being coupled to the pressing lever.

European patent application with publication number EP3584148 discloses a folding device for folding or disassembling rigid objects with engaged toothed rows on opposing surfaces of two connecting plates. Opposite surfaces of protruded teeth of the toothed rows are provided with toothed grooves meshing with each other. When a locking mechanism perpendicular to a direction of the toothed rows is locked, the two connecting plates are driven by the locking mechanism to move toward each other in opposite directions along a pulling rod, so that the toothed grooves on the toothed rows tightly engage with one another, and the two connecting plates are pressed tightly and fixedly connected. When the folding device is in a connected state, meshing surfaces of the two connecting plates tightly mesh with each other through the corresponding toothed grooves, so that the two connected parts are combined together to form a whole rigid combination which can firmly connect a front part and a rear part.

### SUMMARY

An aspect of the present invention is defined by the independent claim below to which reference should now be made. Optional features are defined by the dependent claims.

The object of the utility model is to provide a folding joint for a bicycle, the folding joint being configured to double fasten two closed connecting plates, so that the locking is reliable, the structure is simple, and the manufacturing cost is low.

The folding joint provided in the utility model includes two connecting plates 2 connected to two sections of rods 1 respectively. A side of the two connecting plates 2 are hinged to each other. At least two corresponding edges of opposite cooperating surfaces of the two connecting plates are provided with boss, and each boss of one of the connecting plates is provided with a movable clamping block with a groove on a side thereof. A concave edge of the groove on the side of the clamping block is capable of being in contact with a flange of each boss of the connecting plate simultaneously when the two connecting plates are close together. The clamping block is respectively driven by left and right threads on a screw rod, so that the concave edge of the groove of the clamping block is in contact with or moves away from the flange of the boss of the connecting plate.

According to the folding joint provided in the utility model, the two connecting plates have more than two bosses, when the two connecting plates are closed, the concave edge of the clamping block can be in contact with the flange of the connecting plate boss simultaneously, that is, the two closed connecting plates are clamped so that they cannot be opened. The double locking folding joint uses the left and right threads of the screw rod to drive the movement of the clamping block. The locking or unlocking of the two connecting plates can be achieved by using only the thread of the screw rod to drive a short-distance linear movement of the clamping block. The structure of using the screw rod to control the short-distance movement of the clamping block is very simple, so that the opening and closing operation of the entire folding joint is quick and convenient.

### BRIEF DESCRIPTION OF THE DRAWINGS

FIG. 1 is a perspective view of a folding joint in a half-opened state.
FIG. 2 is a profile view of the folding joint in a closing state.
FIG. 3 is a cross-sectional view of the folding joint.
FIG. 4 is a perspective view of a folding joint in a half-opened state.

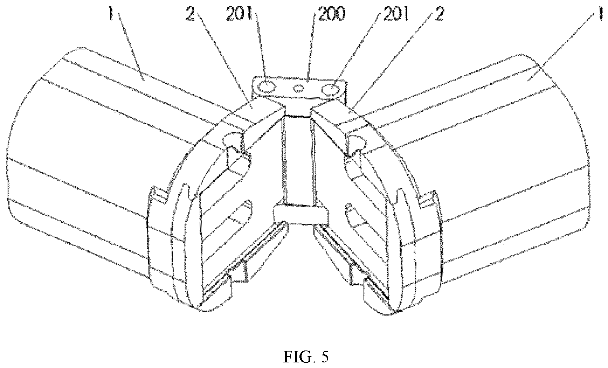

FIG. 5 is a perspective view of a biaxial hinged folding joint in a half-opened state.

### DETAILED DESCRIPTION OF THE EMBODIMENTS

In order to make the above objects, features and advantages of the present utility model clear and easier to understand, the specific embodiments of the present utility model are described in detail below in combination with the accompanying drawings. Many specific details are set forth in the following description to facilitate a full understanding of the present utility model. However, the present utility model can be implemented in many ways different from those described herein, and those skilled in the art can make similar improvements without departing from the connotation of the present utility model. Therefore, the present utility model is not limited by the specific embodiments disclosed below.

In the description of the present utility model, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential direction" are based on the azimuth or position relationship shown in the attached drawings, which are only for the convenience of describing the present utility model and simplifying the description, rather than indicating or implying that the device or element must have a specific azimuth, be constructed and operated in a specific azimuth, so such terms cannot be understood as a limitation of the present utility model.

In addition, the terms "first" and "second" are only used for descriptive purposes and cannot be understood as indicating or implying relative importance or implicitly indicating the number of indicated technical features. Thus, the features defined with "first" and "second" may explicitly or implicitly include at least one of the features. In the description of the present utility model, "multiple" means at least two, such as two, three, etc., unless otherwise expressly and specifically defined.

In the present utility model, unless otherwise expressly specified and limited, the terms "mount", "connect", "contact", "fix" and other terms should be understood in a broad sense, for example, they can be fixed connections, removable connections, or integrated. They can be mechanical connection or electrical connection. They can be directly connected or indirectly connected through an intermediate medium. They can be the connection within two elements or the interaction relationship between two elements, unless otherwise expressly limited. For those skilled in the art, the specific meaning of the above terms in the present utility model can be understood according to the specific situation.

In the present utility model, unless otherwise expressly specified and limited, the first feature "above" or "below" the second feature may be in direct contact with the first and second features, or the first and second features may be in indirect contact through an intermediate medium. Moreover, the first feature is "above" the second feature, but the first feature is directly above or diagonally above the second feature, or it only means that the horizontal height of the first feature is higher than the second feature. The first feature is "below" of the second feature, which can mean that the first feature is directly below or obliquely below the second feature, or simply that the horizontal height of the first feature is less than that of the second feature.

It should be noted that when an element is called "fixed to" or "provided on" another element, it can be directly on another element or there can be a centered element. When an element is considered to be "connected" to another element, it can be directly connected to another element or there may be intermediate elements at the same time. The terms "vertical", "horizontal", "up", "down", "left", "right" and similar expressions used herein are for the purpose of illustration only and do not represent the only embodiment.

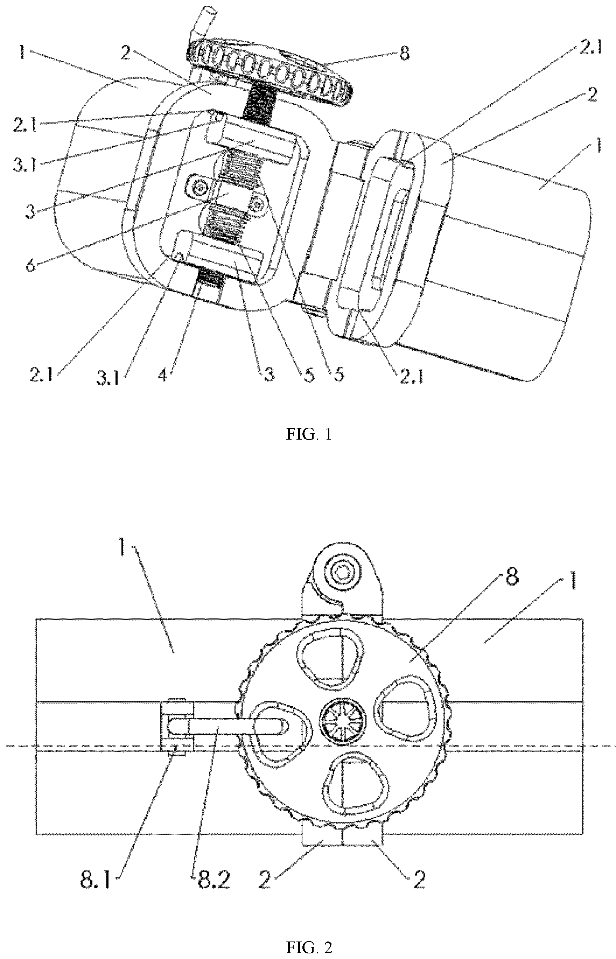

Embodiment 1 is shown in FIGS. 1 to 3.

A folding joint includes two connecting plates 2 connected to two sections of rods 1, respectively. Sides of the two connecting plates 2 are hinged to each other, and opposite cooperating surfaces of the two connecting plates 2 are provided with an upper trapezoid boss 2.1 and a lower trapezoid boss 2.1 inside the two connecting plates 2. Movable clamping blocks 3 are arranged at the upper and lower bosses of one connecting plate 2, and a side of the clamping block 3 adjacent to the trapezoidal boss 2.1 is provided with a trapezoidal groove. An upper section of a screw rod 4 has a right-handed thread connected to a right-hand thread of the upper clamping block 3, and a lower section of the screw rod 4 has a left-handed thread connected to a left-hand thread of the lower clamping block 3. An upper section of screw rod 4 is fixedly connected to a handle, and the screw rod 4 is fixedly connected to the connecting plate 2 through a screw rod buckle 6.

According to the folding joint, the boss of connecting plate 2 can be arranged inside or outside the connecting plate. The two connecting plates 2 are hinged through a biaxial connecting block 200 and two hinge shafts 201. A flange of the boss 2.1 of the connecting plate 2 is trapezoidal or arc-shaped. The rod 1 is provided with a safety buckle seat 8.1 and a safety buckle 8.2.

When the handle 8 is rotated rightwards, the right-handed thread of the screw rod 4 drives the upper clamping block 3 to move upward, so that a concave edge of the trapezoidal groove 3.1 of the upper clamping block 3 is in contact with the flanges of the upper trapezoidal boss 2.1 of the two connecting blocks, respectively, and the lower clamping block 3 is driven to move downward, so that the concave edge of the trapezoidal groove 3.1 of the lower clamping block 3 is in contact with the flanges of the lower trapezoidal boss 2.1 of the two connecting blocks, respectively, which is in a locked state. The two clamping blocks are used to lock the two connecting plates up and down, which is very firm. The clamping block 3 adopts the groove 3.1 to fasten the boss 2.1 of the connecting plate 2, which has a compact structure. The movement of the clamping block 3 is driven by the most common screw rod 4, which has a very simple and reliable structure, so that the connecting plate 2 can become more compact and have good appearance, and the manufacturing process is simple and the cost is low. The safety buckle 8.2 is rotated to insert into a hole of handle 8, so that the handle 8 is locked and cannot rotate, which can prevent the screw rod 4 from rotating to the left accidentally and causing the clamping block 3 to move, which plays a safe role. When the safety buckle 8.2 is rotated out of the hole of the handle 8, and the handle 8 is rotated leftwards, the right-handed thread of the screw rod 4 drives the upper clamping block 3 to move down, so that the concave edge of the trapezoidal groove 3.1 of the upper clamping block 3 is separated from the flanges of the upper trapezoidal boss 2.1 of the two connecting blocks, and the lower clamping block 3 is driven to moved upward, so that the concave edge of the trapezoidal groove 3.1 of the lower clamping block 3 is separated from the flanges of the lower trapezoidal boss 2.1 of the two connecting blocks, respectively, and the two connecting plates can be opened.

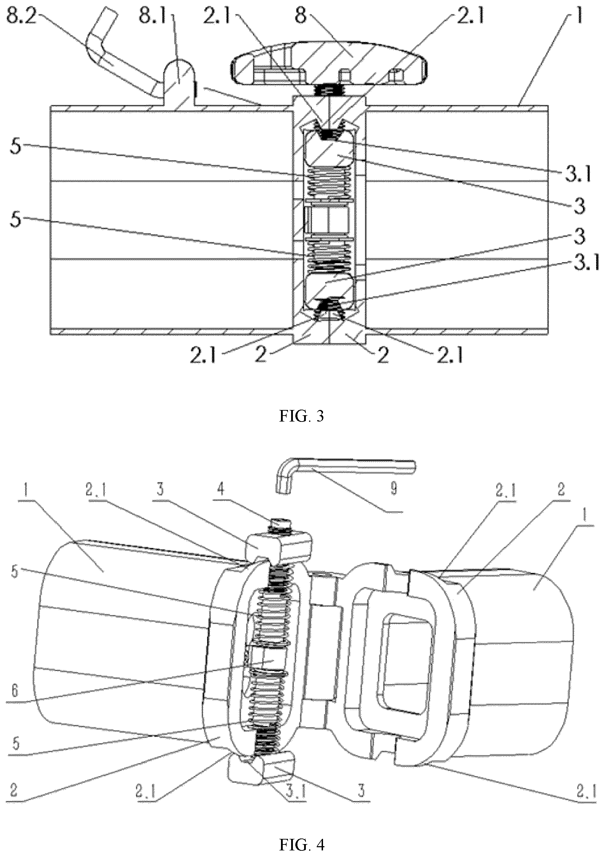

Embodiment 2 is shown in FIG. 4.

A folding joint includes two connecting plates 2 connected to two sections of rods 1, respectively. Sides of the two connecting plates 2 are hinged to each other, and opposite cooperating surfaces of the two connecting plates 2 are provided with an upper trapezoid boss 2.1 and a lower trapezoid boss 2.1 outside the two connecting plates 2. Movable clamping blocks 3 are arranged at the upper and lower bosses of one connecting plate 2, and a side of the clamping block 3 adjacent to the trapezoidal boss 2.1 is provided with a trapezoidal groove. An upper section of a screw rod 4 has a right-handed thread connected to a right-hand thread of the lower clamping block 3, and a lower section of the screw rod 4 has a left-handed thread connected to a left-hand thread of the lower clamping block 3. An upper section of screw rod 4 is provided with an inner hexagonal hole, and the screw rod 4 is fixedly connected to the connecting plate 2 through a screw rod buckle 6.

According to the folding joint, the screw rod 4 is driven to rotate using an external angle wrench or a socket wrench, such as a hexagonal wrench 9. When the screw rod 4 is rotated rightwards by the hexagonal wrench 9, the right-handed thread of the screw rod 4 drives the upper clamping block 3 to move upward, so that the concave edge of the trapezoidal groove 3.1 of the lower clamping block 3 is in contact with the flanges of the lower trapezoidal boss 2.1 of the two connecting blocks, respectively, which is in a locked state. A spring 5 is provided on the screw rod, and the spring 5 keeps pressing the clamping block to prevent the screw rod 4 from rotating leftwards accidentally and causing the clamping block 3 to move, which plays a safe role. When the screw rod 4 is rotated leftwards by the hexagonal wrench 9, the right-handed thread of the screw rod 4 drives the upper clamping block 3 to move down, so that the concave edge of the trapezoidal groove 3.1 of the upper clamping block 3 is separated from the flanges of the upper trapezoidal boss 2.1 of the two connecting blocks, and the lower clamping block 3 is driven to moved upward, so that the concave edge of the trapezoidal groove 3.1 of the lower clamping block 3 is separated from the flanges of the lower trapezoidal boss 2.1 of the two connecting blocks, respectively, and the two connecting plates can be opened.

## Claims

1. A folding joint for a folding bicycle, comprising two connecting plates (2) connected to two sections of rods (1) respectively, sides of the two connecting plates (2) being hinged to each other, wherein two corresponding edges of opposite cooperating surfaces of the two connecting plates (2) are provided with an upper boss (2.1) and a lower boss (2.1), respectively, a movable upper clamping block (3) and a movable lower clamping block (3) each with a groove (3.1) on a side thereof are arranged at the upper boss (2.1) and the lower boss (2.1) of one of the connecting plates (2), respectively; when the two connecting plates (2) are close together, a concave edge of the groove (3.1) on the side of the upper clamping block (3) is capable of being in contact with a flange of the upper boss (2.1) of each connecting plate (2) simultaneously, and a concave edge of the groove (3.1) on the side of the lower clamping block (3) is capable of being in contact with a flange of the lower boss (2.1) of each connecting plate (2) simultaneously;
wherein the folding joint further comprises a screw rod (4), an upper section of the screw rod (4) has a first thread connected to a thread of the upper clamping block (3), a lower section of the screw rod (4) has a second thread connected to a thread of the lower clamping block (3), an upper end of screw rod (4) is connected to a handle (8), and a helical direction of the second thread is opposite to that of the first thread;
when the handle is rotated in a first direction, the first thread of the screw rod (4) drives the upper clamping block (3) to move upward, so that the concave edge of the groove (3.1) of the upper clamping block (3) is in contact with the flange of the upper boss (2.1) of each connecting plate (2), and the lower clamping block (3) is driven to move downward, so that the concave edge of the groove (3.1) of the lower clamping block (3) is in contact with the flange of the lower boss (2.1) of each connecting plate (2);
when the handle is rotated in a second direction, the first thread of the screw rod (4) drives the upper clamping block (3) to move down, so that the concave edge of the groove (3.1) of the upper clamping block (3) is separated from the flange of the upper boss (2.1) of the each connecting plate (2), and the lower clamping block (3) is driven to moved upward, so that the concave edge of the groove (3.1) of the lower clamping block (3) is separated from the flange of the lower boss (2.1) of each connecting plate (2), wherein the second direction is opposite to the first direction.

2. The folding joint according to claim 1, wherein the upper boss (2.1) and the lower boss (2.1) of each connecting plate (2) are arranged inside of the connecting plate (2).

3. The folding joint according to claim 2, wherein the flange of the upper boss (2.1) and the lower boss (2.1) of each connecting plate (2) is trapezoidal or arc-shaped.

4. The folding joint according to claim 3, wherein the two connecting plates (2) are hinged through a biaxial connecting block (200) and two hinge shafts (201).

5. The folding joint according to claim 1, wherein the first thread is a right-handed thread, the upper clamping block (3) has a right-hand thread, the second thread is a left-handed thread, and the lower clamping block (3) has a left-hand thread, the first direction is right-handed, and the second direction is left-handed.

6. The folding joint according to claim 5, wherein a spring (5) is provided on the screw rod (4) to press the clamping block (3).

7. The folding joint according to claim 1, wherein one of the rods (1) is provided with a safety buckle seat (8.1), the folding joint further comprises a safety buckle (8.2) rotatably connected to the safety buckle seat (8.1), and the safety buckle (8.2) is configured to be rotated to insert into a hole of handle (8), so that the handle (8) is locked and cannot rotate.

## Patentansprüche

1. Faltgelenk für ein faltbares Fahrrad, umfassend zwei Verbindungsplatten (2), die jeweils mit zwei Stangenabschnitten (1) verbunden sind, wobei die Seiten der beiden Verbindungsplatten (2) gelenkig miteinander verbunden sind, wobei zwei entsprechende Kanten gegenüberliegender zusammenwirkender Flächen der beiden Verbindungsplatten (2) mit einem oberen Ansatz (2.1) bzw. einem unteren Ansatz (2.1) versehen sind, wobei ein beweglicher oberer Klemmblock (3) und ein beweglicher unterer Klemmblock (3) mit jeweils einer Nut (3.1) an einer Seite davon an dem oberen Ansatz (2.1) bzw. dem unteren Ansatz (2.1) einer der Verbindungsplatten (2) angeordnet sind;
wenn die beiden Verbindungsplatten (2) nahe beieinander liegen, ist eine konkave Kante der Nut (3.1) an der Seite des oberen Klemmblocks (3) in der Lage, gleichzeitig mit einem Flansch des oberen Ansatzes (2.1) jeder Verbindungsplatte (2) in Kontakt zu stehen, und eine konkave Kante der Nut (3.1) an der Seite des unteren Klemmblocks (3) ist in der Lage, gleichzeitig mit einem Flansch des unteren Ansatzes (2.1) jeder Verbindungsplatte (2) in Kontakt zu stehen;
wobei das Faltgelenk weiter eine Schraubstange (4) umfasst, ein oberer Abschnitt der Schraubstange (4) ein erstes Gewinde aufweist, das mit einem Gewinde des oberen Klemmblocks (3) verbunden ist, ein unterer Abschnitt der Schraubstange (4) ein zweites Gewinde aufweist, das mit einem Gewinde des unteren Klemmblocks (3) verbunden ist, ein oberes Ende der Schraubstange (4) mit einem Griff (8) verbunden ist und eine spiralförmige Richtung des zweiten Gewindes zu der des ersten Gewindes entgegengesetzt ist;
wenn der Griff in eine erste Richtung gedreht wird, treibt das erste Gewinde der Schraubstange (4) den oberen Klemmblock (3) so an, dass er sich nach oben bewegt, sodass die konkave Kante der Nut (3.1) des oberen Klemmblocks (3) mit dem Flansch des oberen Ansatzes (2.1) jeder Verbindungsplatte (2) in Kontakt steht, und der untere Klemmblock (3) wird so angetrieben, dass er sich nach unten bewegt, sodass die konkave Kante der Nut (3.1) des unteren Klemmblocks (3) mit dem Flansch des unteren Ansatzes (2.1) jeder Verbindungsplatte (2) in Kontakt steht;
wenn der Griff in eine zweite Richtung gedreht wird, treibt das erste Gewinde der Schraubstange (4) den oberen Klemmblock (3) so an, dass er sich nach unten bewegt, sodass die konkave Kante der Nut (3.1) des oberen Klemmblocks (3) von dem Flansch des oberen Ansatzes (2.1) der jeweiligen Verbindungsplatte (2) getrennt wird, und der untere Klemmblock (3) wird so angetrieben, dass er sich nach oben bewegt, sodass die konkave Kante der Nut (3.1) des unteren Klemmblocks (3) von dem Flansch des unteren Ansatzes (2.1) jeder Verbindungsplatte (2) getrennt wird, wobei die zweite Richtung der ersten Richtung entgegengesetzt ist.

2. Faltgelenk nach Anspruch 1, wobei der obere Ansatz (2.1) und der untere Ansatz (2.1) jeder Verbindungsplatte (2) innerhalb der Verbindungsplatte (2) angeordnet sind.

3. Faltgelenk nach Anspruch 2, wobei der Flansch des oberen Ansatzes (2.1) und des unteren Ansatzes (2.1) jeder Verbindungsplatte (2) trapezförmig oder bogenförmig ist.

4. Faltgelenk nach Anspruch 3, wobei die beiden Verbindungsplatten (2) durch einen biaxialen Verbindungsblock (200) und zwei Scharnierwellen (201) gelenkig miteinander verbunden sind.

5. Faltgelenk nach Anspruch 1, wobei das erste Gewinde ein Rechtsgewinde ist, der obere Klemmblock (3) ein Rechtsgewinde aufweist, das zweite Gewinde ein Linksgewinde ist und der untere Klemmblock (3) ein Linksgewinde aufweist, die erste Richtung rechtsdrehend ist und die zweite Richtung linksdrehend ist.

6. Faltgelenk nach Anspruch 5, wobei eine Feder (5) an der Schraubstange (4) zum Drücken des Klemmblocks (3) bereitgestellt ist.

7. Faltgelenk nach Anspruch 1, wobei eine der Stangen (1) mit einem Sicherheitsschnallensitz (8.1) versehen ist, das Faltgelenk weiter eine Sicherheitsschnalle (8.2) umfasst, die drehbar mit dem Sicherheitsschnallensitz (8.1) verbunden ist, und die Sicherheitsschnalle (8.2) so ausgebildet ist, dass sie zum Einsetzen in ein Loch des Griffs (8) gedreht werden kann, sodass der Griff (8) verriegelt ist und sich nicht drehen kann.

## Revendications

1. Articulation de pliage pour vélo pliant, comprenant deux plaques de liaison (2) reliées respectivement à deux sections de tiges (1), des côtés des deux plaques de liaison (2) étant articulés entre eux, dans laquelle deux bords correspondants de surfaces coopérantes opposées des deux plaques de liaison (2) sont munis respectivement d'un bossage (2.1) supérieur et d'un bossage (2.1) inférieur, un bloc de serrage (3) supérieur mobile et un bloc de serrage (3) inférieur mobile avec chacun une rainure (3.1) sur un côté de ceux-ci sont agencés respectivement au niveau du bossage (2.1) supérieur et du bossage (2.1) inférieur d'une des plaques de liaison (2) ; lorsque les deux plaques de liaison (2) sont proches l'une de l'autre, un bord concave de la rainure (3.1) sur le côté du bloc de serrage (3) supérieur est apte à être en contact simultanément avec une bride du bossage (2.1) supérieur de chaque plaque de liaison (2), et un bord concave de la rainure (3.1) sur le côté du bloc de serrage (3) inférieur est apte à être en contact simultanément avec une bride du bossage (2.1) inférieur de chaque plaque de liaison (2) ;
dans laquelle l'articulation de pliage comprend en outre une tige filetée (4), une section supérieure de la tige filetée (4) présente un premier filetage relié à un filetage du bloc de serrage (3) supérieur, une section inférieure de la tige filetée (4) présente un second filetage relié à un filetage du bloc de serrage (3) inférieur, une extrémité supérieure de la tige filetée (4) est reliée à une poignée (8), et une direction hélicoïdale du second filetage est opposée à celle du premier filetage ;
lorsque la poignée est tournée dans une première direction, le premier filetage de la tige filetée (4) entraîne le déplacement vers le haut du bloc de serrage (3) supérieur, de sorte que le bord concave de la rainure (3.1) du bloc de serrage (3) supérieur soit en contact avec la bride du bossage (2.1) supérieur de chaque plaque de liaison (2), et le bloc de serrage (3) inférieur est entraîné pour se déplacer vers le bas, de sorte que le bord concave de la rainure (3.1) du bloc de serrage (3) inférieur soit en contact avec la bride du bossage (2.1) inférieur de chaque plaque de liaison (2) ;
lorsque la poignée est tournée dans une seconde direction, le premier filetage de la tige filetée (4) entraîne le déplacement vers le bas du bloc de serrage (3) supérieur, de sorte que le bord concave de la rainure (3.1) du bloc de serrage (3) supérieur soit séparé de la bride du bossage (2.1) supérieur de chaque plaque de liaison (2), et le bloc de serrage (3) inférieur est entraîné pour être déplacé vers le haut, de sorte que le bord concave de la rainure (3.1) du bloc de serrage (3) inférieur soit séparé de la bride du bossage (2.1) inférieur de chaque plaque de liaison (2), dans laquelle la seconde direction est opposée à la première direction.

2. Articulation de pliage selon la revendication 1, dans laquelle le bossage (2.1) supérieur et le bossage (2.1) inférieur de chaque plaque de liaison (2) sont agencés à l'intérieur de la plaque de liaison (2).

3. Articulation de pliage selon la revendication 2, dans laquelle la bride du bossage (2.1) supérieur et du bossage (2.1) inférieur de chaque plaque de liaison (2) est trapézoïdale ou arquée.

4. Articulation de pliage selon la revendication 3, dans laquelle les deux plaques de liaison (2) sont articulées à travers un bloc de liaison biaxial (200) et deux arbres d'articulation (201).

5. Articulation de pliage selon la revendication 1, dans laquelle le premier filetage est un filetage à droite, le bloc de serrage (3) supérieur présente un filetage à droite, le second filetage est un filetage à gauche, et le bloc de serrage (3) inférieur présente un filetage à gauche, la première direction est vers la droite, et la seconde direction est vers la gauche.

6. Articulation de pliage selon la revendication 5, dans laquelle un ressort (5) est prévu sur la tige filetée (4) pour presser le bloc de serrage (3).

7. Articulation de pliage selon la revendication 1, dans laquelle l'une des tiges (1) est munie d'un siège de boucle de sécurité (8.1), l'articulation de pliage comprend en outre une boucle de sécurité (8.2) reliée en rotation au siège de boucle de sécurité (8.1), et la boucle de sécurité (8.2) est configurée pour être tournée pour être insérée dans un trou de la poignée (8), de sorte que la poignée (8) soit verrouillée et ne puisse pas tourner.
